(19) Europäisches Patentamt
European Patent Office
Office européen des brevets

(11) EP 1 815 857 A1

(12) EUROPEAN PATENT APPLICATION

(43) Date of publication:
**08.08.2007 Bulletin 2007/32**

(21) Application number: **06002145.8**

(22) Date of filing: **02.02.2006**

(51) Int Cl.:
***A61K 31/404*** *(2006.01)* ***A61K 9/36*** *(2006.01)*
***A61K 47/26*** *(2006.01)* ***A61P 9/00*** *(2006.01)*

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicant: **LEK Pharmaceuticals d.d.**
**1526 Ljubljana (SI)**

(72) Inventors:
- **Kovacic, Mateja**
  **1000 Ljubljana (SI)**
- **Husu-Kovacevic, Breda**
  **1000 Ljubljana (SI)**

(74) Representative: **Dietz, Jörg-Reimar et al**
**Novartis AG,**
**Corporate Intellectual Property**
**4002 Basel (CH)**

# (54) A pharmaceutical composition comprising perindopril

(57) The present invention provides a stable pharmaceutical composition comprising an inclusion complex of perindopril, a microcrystalline cellulose having a low moisture content and/or a silicified microcrystalline cellulose having a low moisture content, and optionally other pharmaceutically acceptable excipients.

EP 1 815 857 A1

## Description

The present invention relates to a stable pharmaceutical composition of the ACE inhibitor perindopril, or a salt, or an addition salt or a derivative thereof.

Perindopril and its pharmaceutically acceptable salts are known as angiotensin converting enzyme inhibitors and are used in the treatment of cardiovascular diseases, especially in the treatment of hypertension and heart failure. Perindopril is chemically known as (2S,3aS,7aS)-((2-(1-(ethoxycarbonyl)-(S)-butylamino)-(S)-propionyl)octahydro-indole-2-carboxylic acid and can be represented by formula (I).

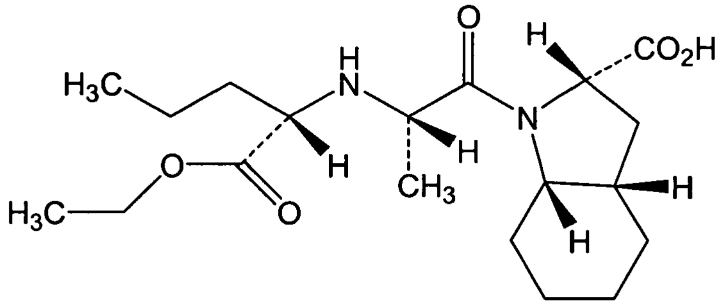

**(I)**

Perindopril is known, for example, from EP-A 0049658; the tert-butylamine salt thereof, i.e. perindopril erbumine, is known from EP-A 0308 341.

EP 1296947 B1, EP 1294689 A1, EP 1296948 B1 disclose α, β and γ crystalline forms of perindopril erbumine, respectively, and WO 2004/113293 discloses δ and ε crystalline form of perindopril erbumine.

It is known that ACE inhibitors are susceptible to degradation via a) hydrolysis of the side-chain ester group, b) intramolecular cyclization to form diketopiperazines, c) isomerisation at some chiral centres and d) oxidation to form coulored products. Perindopril is especially susceptible to hydrolysis and to intramolecular cyclization due to its molecular structure.

The main degradation products of perindopril are diketopiperazine (ethyl (2S)-2-[(3S,5aS,9aS,10aS)-3-methyl-1,4-dioxodecahydropyrazino[1,2-a]indol-2(1H)-yl]pentanoate), known as impurity F in European Pharmacopea 5.0, obtained after intramolecular cyclization, and perindoprilate ((2S,3aS,7aS)-1-[(2S)-2-[[(1S)-1-carboxybutyl]amino]propanoyl]octahydro-1H-indole-2-carboxylic acid), known as impurity B in European Pharmacopea 5.0, obtained after hydrolysis of side-chain ester group.

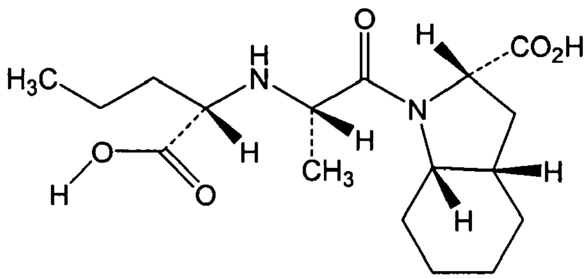

Impurity B

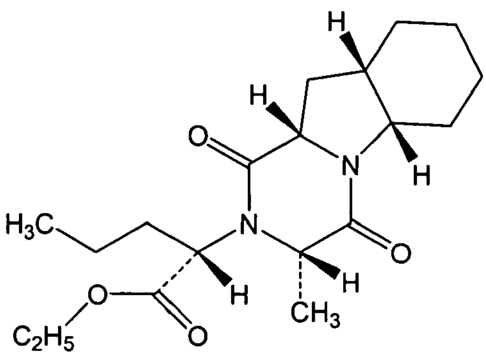

Impurity F

Different methods of stabilizing ACE inhibitors in pharmaceutical compositions are known in the prior art. For example, pharmaceutical compositions comprising ACE inhibitors can be stabilized by the presence of alkali or alkaline metal salts (WO 01/15724, EP 280 999), magnesium oxide (WO 99/62560), hydrochloric acid donors (EP 468 929), ascorbic acid (EP 264 888).

Furthermore, effects of different pharmaceutical excipients on the stability of ACE inhibitors have been also disclosed in the prior art.

GB 2 394 660 discloses that the presence of colloidal silicon dioxide promotes the degradation of ACE inhibitors in pharmaceutical compositions.

WO 2005/094793 describes a solid pharmaceutical composition comprising perindopril or a salt thereof, microcrystalline cellulose having a low moisture content, anhydrous lactose, and inorganic carbonate, and process for the preparation thereof using dry mixing method.

According to WO 2005/068490 the stability of perindopril is increased by formation of inclusion complexes with cyclodextrins or their alkylated and hydroxyalkylated derivatives or by formation of complexes with water-soluble polymers such as polyvinylpyrrolidone and hydroxyalkylcellulose derivatives.

However, the problem of the stability of pharmaceutical composition comprising ACE inhibitors has not been solved completely yet. Therefore, there is still a need to develop a stable pharmaceutical composition comprising ACE inhibitors, particularly perindopril, a salt, an addition salt or a derivative thereof.

Therefore, the present invention relates to stable pharmaceutical compositions comprising an inclusion complex of perindopril, or a salt, or an addition salt or a derivative thereof with hydroxypropyl-β-cyclodextrin, wherein said pharmaceutical compositions is substantially free of lactose.

Commercially available compositions of perindopril erbumine comprise crystalline perindopril erbumine and conventional excipients, such as microcrystalline cellulose, lactose monohydrate, hydrophobic colloidal silica, magnesium stearate.

It was found that the use of perindopril erbumine inclusion complexes with cyclodextrins or alkylated and hydroxyalkylated derivative thereof instead of crystalline perindopril erbumine for the preparation of a pharmaceutical composition results in decreasing of the formation of diketopiperazines during the storage. Furthermore, it was surprisingly found that the lowest percent of diketopiperazines is obtained when an inclusion complex of perindopril erbumine with hydroxypropyl-β-cyclodextrin is used for the preparation of a pharmaceutical composition.

Additionally, a pharmaceutical composition of the present invention, wherein perindopril erbumine included in an inclusion complex is in amorphous form avoids a transformation of polymorphs of perindopril erbumine, which may occur in a pharmaceutical composition comprising crystalline perindopril erbumine.

Furthermore, it was surprisingly found that various excipients used in the preparation of a pharmaceutical composition of the present invention do not have a significant effect on the formation of diketopiperazines during the storage.

However, it was surprisingly found that hydroxypropyl-β-cyclodextrin inclusion complex of perindopril erbumine is not compatible with lactose, which is usually used for the preparation of pharmaceutical compositions comprising crystalline perindopril erbumine and was also comprised in a pharmaceutical composition comprising an inclusion complex of perindopril erbumine with β-cyclodextrin disclosed in WO 2005/068490. Use of lactose for the preparation of a pharmaceutical composition comprising inclusion complex of perindopril erbumine with hydroxypropyl-β-cyclodextrin results in increasing of the formation of impurities other than diketopiperazines during the storage.

Therefore, the first embodiment of the present invention relates to a lactose-free pharmaceutical composition comprising an inclusion complex of perindopril, or a salt, or an addition salt or a derivative thereof with hydroxypropyl-β-cyclodextrin

Preferably, a pharmaceutical composition of the present invention comprises perindopril erbumine inclusion complex with hydroxypropyl-β-cyclodextrin.

Additionally, it was found that the use of a microcrystalline cellulose having a low moisture content as filler, results in improved stability of pharmaceutical composition comprising an inclusion complex of perindopril erbumine with hydroxypropyl-β-cyclodextrin.

The term "microcrystalline cellulose having a low moisture content" means a microcrystalline cellulose having a moisture content of less than 3% (w/w) determined by Karl Fischer titration method.

Commercially available specialized grade microcrystalline cellulose having a low moisture content, e.g. Avicel® PH 112, Avicel® PH 113, or pre-dried microcrystalline cellulose may be used in the preparation of a pharmaceutical composition of the present invention.

Preferably a microcrystalline cellulose having a low moisture content is a microcrystalline cellulose having a moisture content less than 2 % (w/w), more preferably a microcrystalline cellulose having a moisture content less than 1.5 % (w/w), most preferably a microcrystalline cellulose having a moisture content less than 0.5 % (w/w).

Further, in contrast to the teaching of GB 2 394 660 that the presence of colloidal silicone dioxide promotes the degradation of ACE inhibitors it was surprisingly found that also replacement of lactose with a silicified microcrystalline cellulose having a low moisture content results in improved stability of pharmaceutical composition comprising an inclusion complex of perindopril erbumine with hydroxypropyl-β-cyclodextrin.

The term "silicified microcrystalline cellulose having a low moisture content" means a silicified microcrystalline cellulose having a moisture content of less than 4% (w/w) determined by Karl Fischer titration method.

Commercially available silicified microcrystalline cellulose having a low moisture content, e.g. Prosolv SMCC50® LM, or pre-dried silicified microcrystalline cellulose may be used in the preparation of a pharmaceutical composition of

the present invention.

Preferably a silicified microcrystalline cellulose having a low moisture content is a silicified microcrystalline cellulose having a moisture content less than 3 % (w/w), more preferably a silicified microcrystalline cellulose having a moisture content less than 1.5 % (w/w), most preferably a silicified microcrystalline cellulose having a moisture content less than 0.5 % (w/w).

Furthermore, the use of silicified microcrystalline cellulose having improved compaction properties compared to conventional microcrystalline cellulose contributes to better compressibility of direct compression compositions. Silicified microcrystalline cellulose has greater tensile strength and requires lower compression force and enables manufacture of tablets having short disintegration time.

Therefore, another embodiment of the present invention is related to a lactose-free pharmaceutical composition comprising:

(a) an inclusion complex of perindopril, or a salt, or an addition salt or a derivative thereof with hydroxypropyl-β-cyclodextrin, preferably an inclusion complex of perindopril erbumine with hydroxypropyl-β-cyclodextrin,
(b) at least one of filler selected from the group consisting of a microcrystalline cellulose having a moisture content less than 3 % (w/w) and a silicified microcrystalline cellulose having a moisture content less than 4 % (w/w).

Some additional pharmaceutical excipients can be added into the pharmaceutical composition of the present invention in order to improve its technological properties like powder flowability and compressibility of the dry mixture containing active ingredient and excipients and to attain the desired release rate of perindopril erbumine from pharmaceutical composition.

Pharmaceutical composition of the present invention may contain one or more additional pharmaceutical excipients such as binders, disintegrants, glidants, lubricants, etc.

Suitable binder may be selected from the group consisting of starch, e.g. Starch 1500® LM, pregelatinized starch, gelatine, sodium carboxymethylcellulose, polyvinylpyrrolidone, alginic acid, sodium alginate, acacia, carbomer, dextrin, guar gum, hydrogenated vegetable oil, methylcellulose, ehylcellulose, hydroxyethylcellulose, hydroxypropylcellulose, hydroxypropylmethylcellulose, glucose syrup, magnesium aluminium silicate, maltodextrin, polymethacrylates, zein.

Suitable disintegrant may be selected from the group consisting of starch, pregelatinized starch, sodium starch glycolate, sodium carboxymethylcellulose, cross-linked sodium carboxymrethylcellulose, calcium carboxymethylcellulose, methylcellulose, powdered cellulose, silicified microcrystalline cellulose, polacrilin potassium, e.g. Amberlite®, cross-linked polivinylpyrrolidone, alginic acid, sodium alginate, colloidal silicon dioxide, guar gum, magnesium aluminium silicate, and others. Preferred disintegrants are silicified microcrystalline cellulose and polacrilin potassium.

Suitable glidant may be selected from the group consisting of magnesium stearate, calcium stearate, aluminium stearate, stearic acid, palmitic acid, cetanol, stearol, polyethylene glycols of different molecular weights, magnesium trisilicate, calcium phosphate, colloidal silicon dioxide, e.g. Aerosil®, micronized silicon dioxide, e.g. Syloid® AL-1, talc, powdered cellulose, starch and others. Preferred glidants are colloidal silicon dioxide and micronized silicon dioxide.

Suitable lubricant may be selected from the group consisting of stearic acid, calcium, magnesium, zinc or aluminium stearate, siliconized talc, glycerol monostearate, glycerol palmitostearate, hydrogenated castor oil, hydrogenated vegetable oil, mineral oil, light mineral oil, polyethylene glycol, sodium benzoate, sodium lauryl sulphate, sodium stearyl fumarate, talc and others. Preferred lubricant is magnesium stearate.

Preferred lactose-free pharmaceutical composition of the present invention comprises:

(a) 1-25 % (w/w) of an inclusion complex of perindopril erbumine with hydroxypropyl-β-cydodextrin,
(b) 0-30 % (w/w) of microcrystalline cellulose having a moisture content less than 3 % (w/w),
(c) 0-60 % (w/w) of silicified microcrystalline cellulose having a moisture content less than 4 % (w/w),
(d) 0.5-2 % (w/w) of one or more disintegrants,
(e) 0.5-4 % (w/w) of one or more glidants, and
(f) 0.5-2% (w/w) of one or more lubricants.

More preferred lactose-free pharmaceutical composition of the present invention comprises:

(a) 1-25 % (w/w) of an inclusion complex of perindopril erbumine with hydroxypropyl-β-cyclodextrin,
(b) 0-30 % (w/w) of microcrystalline cellulose having a moisture content less than 3 % (w/w),
(c) 0-60 % (w/w) of silicified microcrystalline cellulose having a moisture content less than 4 % (w/w),
(d) 0.5-2% (w/w) of polacrilin potassium,
(e) 0-2 % (w/w) of colloidal silicon dioxide,
(f) 0.5-2% (w/w) of micronized silicon dioxide, and

(g) 0.5-2% (w/w) of magnesium stearate.

The microcrystalline cellulose having a moisture content less than 3 % (w/w) is preferably a microcrystalline cellulose having a moisture content less than 2 % (w/w), more preferably a microcrystalline cellulose having a moisture content less than 1.5 % (w/w), most preferably a microcrystalline cellulose having a moisture content less than 0.5 % (w/w). The silicified microcrystalline cellulose having a moisture content less than 4 % (w/w) is preferably a silicified microcrystalline cellulose having a moisture content less than 3 % (w/w), more preferably a silicified microcrystalline cellulose having a moisture content less than 1.5 % (w/w), most preferably a silicified microcrystalline cellulose having a moisture content less than 0.5 % (w/w).

A pharmaceutical composition of the present invention may be used for the preparation of a medicament for use in the treatment of cardiovascular diseases, e.g. hypertension or heart failure.

A pharmaceutical composition of the present invention comprises a therapeutically effective amount of an inclusion complex of perindopril, or a salt, or an addition salt or a derivative thereof with hydroxypropyl-β-cydodextrin, preferably a therapeutically effective amount of an inclusion complex of perindopril erbumine with hydroxypropyl-β-cyclodextrin.

A therapeutically effective amount of such complex is the amount of the complex which comprises an amount of perindopril erbumine which is appropriate in a dosage form useful to treat cardiovascular diseases, e.g hypertension. In general, a pharmaceutically effective amount of perindopril erbumine is 1 to 15 mg, preferably 2 to 8 mg, more preferably 2, 4 or 8 mg.

A pharmaceutical compositions according to the present invention is preferably in solid form, including tablets, capsules, caplets, lozenges and sachets. Tablets may be suitably coated (film coated tablets, pills). Capsule formulations may cover both soft and hard capsules. A pharmaceutical compositions according to the present invention is more preferably in form of tablets.

Further, a pharmaceutical composition of the present invention may be a combination product comprising one or more additional pharmaceutically active components in addition to perindopril, or a salt, or an addition salt or a derivative thereof. Preferably, the additional pharmaceutically active component is selected from the group consisting of a diuretic, β-blocker, a calcium-channel blocker, a vasodilator, an anti- hypertensive drug, or an angiotensin II receptor antagonist. More preferably, the additional pharmaceutically active component is a diuretic. The diuretic may be selected from the group consisting of indaparride, hydrochlorothiazide, furosemide, altizide, trichlormethiazide, triflumethazide, bemetizide, ccyclothiazide, methylclothiazide, azosemide, chlorothiazide, butizide, bendrofluazide, cyclopenthiazide, benzchlortriazide, polythiazide, hydroflumethiazide, benzthiazide, ethiazide, penflutazide, clopamide, cicletanide and piretanide, or a salt, or an addition salt or a derivative thereof. Preferably the diuretic is indapamide or hydrochlorothiazide, more preferably the diuretic is indapamide.

In another option the present invention relates to a lactose-free pharmaceutical composition comprising:

(a) an inclusion complex of perindopril, or a salt, or an addition salt or a derivative thereof with hydroxypropyl-β-cyclodextrin,
(b) indapamide,
(c) at least one of filler selected from the group consisting of a microcrystalline cellulose having a moisture content less than 3 % (w/w) and a silicified microcrystalline cellulose having a moisture content less than 4 % (w/w).

A pharmaceutical composition of the present invention may be prepared by direct compression method, wherein a mixture of an inclusion complex of perindopril erbumine with hydroxypropyl-β-cyclodextrin, a microcrystalline cellulose having a low moisture content and/or a silicified microcrystalline cellulose having a low moisture content and optionally other excipients are dry blended, homogenized and pressed into tablets.

Furthermore, it was found that even more stable pharmaceutical composition is obtained if a pharmaceutical composition of the present invention is prepared under controlled environmental conditions, preferably at relative humidity equal or less than 35 % and at temperature equal or less than 30 °C, more preferably at relative humidity equal or less than 30 % and at temperature equal or less than 25 °C.

Another embodiment of the present is a process for the preparation of the pharmaceutical composition of the present invention comprising steps of:

(a) dry mixing a mixture of:

- an inclusion complex of perindopril erbumine with hydroxypropyl-β-cyclodextrin,
- at least one of filler selected from the group consisting of a microcrystalline cellulose having a moisture content less than 3 % (w/w) and a silicified microcrystalline cellulose having a moisture content less than 4 % (w/w),
- optionally indapamide, and

- optionally other excipients,

(b) pressing the mixture obtained in step (a) into a tablet.

Preferably a process for the preparation of the pharmaceutical composition of the present invention comprising steps of:

(a) dry mixing a mixture of:

- an inclusion complex of perindopril erbumine with hydroxypropyl-β-cyclodextrin,
- at least one of filler selected from the group consisting of a microcrystalline cellulose having a moisture content less than 3 % (w/w) and a silicified microcrystalline cellulose having a moisture content less than 4 % (w/w),
- optionally indapamide, and
- optionally other excipients,

(b) adding the lubricant to the mixture obtained in step (a),
(c) homogenizing the mixture obtained in step (b),
(d) pressing the mixture obtained in step (c) into a tablet.

The microcrystalline cellulose having a moisture content less than 3 % (w/w) is preferably a microcrystalline cellulose having a moisture content less than 2 % (w/w), more preferably a microcrystalline cellulose having a moisture content less than 1.5 % (w/w), most preferably a microcrystalline cellulose having a moisture content less than 0.5 % (w/w). The silicified microcrystalline cellulose having a moisture content less than 4 % (w/w) is preferably a silicified microcrystalline cellulose having a moisture content less than 3 % (w/w), more preferably a silicified microcrystalline cellulose having a moisture content less than 1.5 % (w/w), most preferably a silicified microcrystalline cellulose having a moisture content less than 0.5 % (w/w).

Preferably, the process for the preparation of the pharmaceutical composition of the present invention is performed in an environment of a relative humidity equal or less than 35% and at temperature equal or less than 30 °C, preferably in an environment of a relative humidity equal or less than 30% and at temperature equal or less than 25 °C.

The following examples illustrate the invention, but do not limit it in any way:

Example 1 (Comparative example)

Composition of tablet comprising 4 mg of perindopril erbumine:

| | mg/tablet |
|---|---|
| Perindopril erbumine - crystalline | 4,000 |
| Lactose anhydrous | 36,950 |
| Microcrystalline cellulose (low moisture) | 48,500 |
| Colloidal silicon dioxide | 0.250 |
| Magnesium stearate | 0.300 |

Perindopril erbumine, lactose, cellulose and colloidal silicon dioxide are dry-mixed. Magnesium stearate is added to the obtained blend. The resulting mixture is mixed for a short time and compressed into tablet.

Example 2 (Comparative example)

Composition of tablet comprising 4 mg of perindopril erbumine:

| | mg/tablet |
|---|---|
| Perindopril erbumine - crystalline | 4,000 |
| Silicified microcrystalline cellulose (low moisture) | 42,750 |
| Polacrilin potassium | 0,900 |

(continued)

| | mg/tablet |
|---|---|
| Micronized silicon dioxide | 0,900 |
| Colloidal silicon dioxide | 0,450 |
| Microcrystalline cellulose (low moisture) | 40,100 |
| Magnesium stearate | 0,900 |

Perindopril erbumine, microcrystalline cellulose, silicified microcrystalline cellulose, polacrilin potassium, colloidal silicon dioxide, and micronized silicon dioxide are dry-mixed. Magnesium stearate is added to the obtained blend. The resulting mixture is mixed for a short time and compressed into tablet.

Example 3 (Comparative example)

Composition of tablet comprising 4 mg of perindopril erbumine:

| | mg/tablet |
|---|---|
| Perindopril erbumine - hydroxypropyl-β-cyclodextrin inclusion complex | 20,000 |
| Lactose anhydrous | 28.950 |
| Colloidal silicon dioxide | 0,250 |
| Microcrystalline cellulose (low moisture) | 40,500 |
| Magnesium stearate | 0,300 |

Perindopril erbumine - hydroxypropyl-β-cydodextrin inclusion complex, lactose, microcrystalline cellulose, and colloidal silicon dioxide are dry-mixed. Magnesium stearate is added to the obtained blend. The resulting mixture is mixed for a short time and compressed into tablet.

Example 4 (Invention)

| | mg/tablet |
|---|---|
| Perindopril erbumine - hydroxypropyl-β-cydodextrin inclusion complex | 20,000 |
| Micronized silicon dioxide | 0,900 |
| Colloidal silicon dioxide | 0,450 |
| Microcrystalline cellulose (low moisture) | 67,750 |
| Magnesium stearate | 0,900 |

Perindopril erbumine - hydroxypropyl-β-cyclodextrin inclusion complex, microcrystalline cellulose, colloidal silicon dioxide, and micronized silicon dioxide are dry-mixed. Magnesium stearate is added to the obtained blend. The resulting mixture is mixed for a short time and compressed into tablet.

Example 5 (Invention)

Composition of tablet comprising 4 mg of perindopril erbumine:

| | mg/tablet |
|---|---|
| Perindopril erbumine - hydroxypropyl-β-cyclodextrin inclusion complex | 20,000 |

(continued)

| | mg/tablet |
|---|---|
| Silicified microcrystalline cellulose (low mioisture) | 42,750 |
| Polacrylin potassium | 0,900 |
| Micronized silicon dioxide | 0,900 |
| Colloidal soilicon dioxide | 0,450 |
| Microcrystalline cellulose (low moisture) | 24,100 |
| Magnesium stearate | 0,900 |

Perindopril erbumine - hydroxypropyl-β-cyclodextrin inclusion complex, microcrystalline cellulose, silicified microcrystalline cellulose, polacrilin potassium, colloidal silicon dioxide, and micronized silicon dioxide are dry-mixed. Magnesium stearate is added to the obtained blend. The resulting mixture is mixed for a short time and compressed into tablet.

<u>Example 6 - combination with indapamide (Invention)</u>

Composition of tablet comprising 4 mg of perindopril erbumine and 1.25 mg of indapamide:

| | mg/tablet |
|---|---|
| Perindopril erbumine - hydroxypropyl-β-cyclodextrin inclusion complex | 20,000 |
| Indapamide | 1,250 |
| Silicified microcrystalline cellulose (low mioisture) | 42,750 |
| Polacrylin potassium | 0,900 |
| Micronized silicon dioxide | 0,900 |
| Colloidal soilicon dioxide | 0,450 |
| Microcrystalline cellulose (low moisture) | 22,850 |
| Magnesium stearate | 0,900 |

Perindopril erbumine - hydroxypropyl-β-cyclodextrin inclusion complex, microcrystalline cellulose, silicified microcrystalline cellulose, polacrilin potassium, colloidal silicon dioxide, micronized silicon dioxide, and indapamide are dry-mixed. Magnesium stearate is added to the obtained blend. The resulting mixture is mixed for a short time and compressed into tablet.

<u>Example 7</u>

Stability tests

For the stability studies samples of the comparative and invention compositions of tablets are selected and the results are evaluated together with the results of innovator product Coversyl®.

Samples of different compositions packaged into Alu-Alu blisters and Coversyl® tablets packaged into the original blisters are exposed to the accelerated stability testing at 40°C / 75% relative humidity.

Results are presented in the table 1.

Table 1:

| Results of stability testing | | | | | | |
|---|---|---|---|---|---|---|
| Example No. | Time of testing | Water (%) | Assay (%) | Total impurities (%) | Impurity B (%) | Impurity F (%) |
| 1 | 0 | 2.8 | 97.0 | 0.05 | < 0.05 | < 0.05 |
| | 1month | 3.1 | 98.8 | 0.80 | 0.17 | 0.42 |
| 2 | 0 | 3.9 | 96.7 | 0.07 | / | 0.07 |
| | 1 month | 3.2 | 93.5 | 0.41 | 0.13 | 0.28 |
| | 2 months | 4.5 | 90.2 | 0.90 | 0.29 | 0.49 |
| 3 | 0 | 2.1 | 101.3 | 0.37 | 0.06 | < 0.05 |
| | 1 month | 2.7 | 102.5 | 1.05 | 0.14 | 0.05 |
| 4 | 0 | / | / | 0.24 | 0.07 | <0.05 |
| | 1 month | / | / | 0.31 | 0.18 | 0.06 |
| 5 | 0 | 3.4 | 102.8 | 0.10 | 0.05 | < 0.05 |
| | 1 month | 3.5 | 102.6 | 0.47 | 0.28 | < 0.05 |
| | 3 months | 5.3 | 102.1 | 1.20 | 0.86 | 0.06 |
| | 6 months | 5.3 | 100.0 | 2.78 | 2.07 | 0.12 |
| Coversyl® tablets (8 mg) | 0 | 5.4 | 94.4 | 0.56 | 0.15 | <0.05 |
| | 1 month | / | / | 1.86 | 1.12 | <0.05 |
| | 3 months | 5.3 | / | 6.22 | 4.48 | 0.22 |
| | 6 months | 5.3 | 87.7 | 14.40 | 9.42 | 0.50 |

During the accelerated stability testing following parameters are analyzed with the selected analytical methods presented below:

- water content,
- assay of perindopril,
- related substances/degradation products.

Among test parameters the most significant for the stability is determination of related substances and degradation products.

Stability testing of different perindopril erbumine tablets has shown that:

- the formation of diketopipirazine in tablets comprising perindopril erbumine inclusion complex with hydroxypropyl-β-cyclodextrin is very low,
- the formation of impurities other than diketopipirazine is the highest in the tablets comprising lactose,
- lactose-free tablets comprising microcrystalline cellulose having a low water content and lactose-free tablets comprising microcrystalline cellulose having a low water content and silicified microcrystalline cellulose having a low water content show high stability.

Example 8

Analytical methods

A) Degradation products

Degradation products of perindopril are determined using HPLC method as described in European Pharmacopoeia 5.0 (01/2005, monograph for Perindopril tert-butylamine - pages 2210-2212).

B) Water content

Karl Fischer titration method determined according to the PhEur requirements. 300 mg of tablet powder is determined directly in the methanol titration reagent.

C) Assay

Isocratic HPLC determination with the external standard method Chemicals, Reagents, Standards, Equipment:

| | | |
|---|---|---|
| Working standard | : | Perindopril erbumine ($C_{23}H_{43}N_3O_5$) |
| Water | : | HPLC-grade |
| Acetonitrile (ACNL) | : | HPLC-grade |
| Triethylamine ($Et_3N$) | : | HPLC-grade |
| Methanol, $C_2H_5OH$ | : | HPLC-grade |
| Ortho-Phosphoric acid ($H_3PO_4$) | : | analytical grade (85 %) |
| Solutions | | |
| $NaH_2PO_4$-buffer, pH 2.5 | : | 1.2 g of $NaH_2PO_4$ is dissolved in 1000 ml of water, 3,0 mL of $Et_3N$ is added and the solution is adjusted to pH 2.5 with $H_3PO_4$ using a pH-meter. |
| Solvent | : | methanol: buffer pH 2.5 = 1:1 (v/v) |

Chromatographic Conditions:

| | | |
|---|---|---|
| HPLC apparatus | : | Waters |
| Column | : | Luna C18(2), 3$\mu$m, 150 $\times$ 4.6 mm |
| System | : | isocratic |
| Eluent | : | $NaH_2PO_4$-buffer, pH 2.5 / ACNL = 730 / 270 (v/v) |
| Flow rate | : | 1.0 ml/min |
| Oven temperature | : | 50 °C |
| Injection volume | : | 20 $\mu$l |
| Stop time | : | 10 min |
| Detection | : | $\lambda$ = 205 nm |
| Autosampler | : | 20 °C |

Preparation of the solutions:

Reference stock solution:

50 mg of the perindopril erbumine working standard is dissolved in a 40 mL of solvent in a 50-ml volumetric flask and exposed to ultrasound for 5 minutes, filled up to the mark with solvent and shaked well (solution A). 5.0 mL of the reference stock solution was pipetted into a 25-ml volumetric flask and filled up to the mark with solvent ($C_{perindopril}$ = 0.2 mg/ml). The obtained solution is filtered.

Test solution:

The average mass of the tablet was determined by weighting 20 tablets. The amount of tablet powder corresponding to 40 mg of perindopril erbumine was dissolved in 100 ml of solvent in a 200-ml volumetric flask and exposed to ultrasound for 10 minutes, filled up to the mark with solvent and shaked well. The obtained solution is filtered.

System Suitability Test (SST)

Reproducibility:

With repeated injections of the reference solution ($n \geq 5$), carried out along the entire testing sequence, relative standard deviations (RSD) of not more than 2.0 % must be obtained for the peak area of perindopril erbumine.

Tailing or symmetry factor:

T perindopril erbumine ≤1,5

Plate count:

N perindopril erbumine ≥1000

Calculations:

(1) Calculation of Perindopril erbumine:

$$\frac{P_R * A_T * m * C * RT}{P_T * A_R * 100 * Rs} = \text{\% of Perindopril erbumine}$$

| | | |
|---|---|---|
| $P_T$ | = | initial mass of the tablet powder to be tested in mg |
| $P_R$ | = | initial mass of the perindopril erbumine working standard in the reference solution in mg |
| $A_T$ | = | peak area of perindopril erbumine in the chromatogram of the test solution |
| $A_R$ | = | peak area of perindopril erbumine in the chromatogram of the reference solution |
| m | = | average mass of the tablet in mg |
| C | = | content of perindopril erbumine in the working standard in % |
| RS | = | dilution factor from test solution |
| RT | = | dilution factor from reference stock solution |

## Claims

**1.** A lactose-free pharmaceutical composition comprising an inclusion complex of perindopril, or a salt, or an addition salt or a derivative thereof with hydroxypropyl-β-cyclodextrin,

**2.** A pharmaceutical composition according to claim 1 further comprising:at least one of filler selected from the group consisting of a microcrystalline cellulose having a moisture content less than 3 % (w/w) and a silicified microcrystalline cellulose having a moisture content less than 4 % (w/w).

**3.** A pharmaceutical composition according to claim 1 or claim 2 comprising an inclusion complex of perindopril erbumine with hydroxypropyl-β-cyclodextrin.

**4.** A pharmaceutical composition according to any one of claims 1 to 3 comprising a microcrystalline cellulose having a moisture content less than 2 % (w/w).

**5.** A pharmaceutical composition according to any one of claims 1 to 3 comprising a silicified microcrystalline cellulose having a moisture content less than 3 % (w/w)

**6.** A pharmaceutical composition according to any one of claims 1 to 3 comprising:

(a) 1-25 % (w/w) of inclusion complex of perindopril erbumine with hydroxypropyl-β-cyclodextrin,
(b) 0-30 % microcrystalline cellulose having a moisture content less than 3 % (w/w).
(c) 0-60 % silicified microcrystalline cellulose having a moisture content less than 4 % (w/w),
(g) 0.5-2 % (w/w) of one or more disintegrants,
(h) 0.5-4 % (w/w) of one or more glidants, and
(d) 0.5-2% (w/w) of one or more lubricants.

7. A pharmaceutical composition according to any one of claims 1 to 6 further comprising one or more additional pharmaceutically active components.

8. A pharmaceutical composition according to claim 7, wherein said additional pharmaceutically active component is a diuretic.

9. A pharmaceutical composition according to claim 8, wherein said diuretic is indapamide.

10. A process for the preparation of pharmaceutical composition according to any one of claims 1 to 9 comprising the steps of:

(a) dry mixing a mixture of:

- an inclusion complex of perindopril erbumine with hydroxypropyl-β-cyclodextrin,
- at least one of filler selected from the group consisting of a microcrystalline cellulose having a moisture content less than 3 % (w/w) and a silicified microcrystalline cellulose having a moisture content less than 4 % (w/w) and
- optionally indapamide,
- optionally other excipients,

(b) pressing the mixture obtained in step (a) into a tablet.

11. A process for the preparation of pharmaceutical composition according to any one of claims 1 to 9 comprising the steps of:

(a) dry mixing a mixture of:

- an inclusion complex of perindopril erbumine with hydroxypropyl-β-cyclodextrin,
- at least one of filler selected from the group consisting of a microcrystalline cellulose having a moisture content less than 3 % (w/w) and a silicified microcrystalline cellulose having a moisture content less than 4 % (w/w) and
- optionally indapamide,
- optionally other excipients,

(b) adding the lubricant to the mixture obtained in step (a),
(c) homogenizing the mixture obtained in step (b),
(d) pressing the mixture obtained in step (c) into a tablet.

12. A process according to claim 11 or claim 12 wherein said process is performed in an environment of a relative humidity equal or less than 35% of and at temperature equal or less than 30 °C.

13. Use of the pharmaceutical composition according to any one of claims 1 to 9 for the preparation of a medicament for use in the treatment of cardiovascular diseases.

European Patent Office

# EUROPEAN SEARCH REPORT

Application Number
EP 06 00 2145

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,X | WO 2005/068490 A (LEK PHARMACEUTICALS D.D; RUCMAN, RUDOLF) 28 July 2005 (2005-07-28)<br>* abstract *<br>* examples 5,6 *<br>* claims *<br>----- | 1-13 | A61K31/404<br>A61K9/36<br>A61K47/26<br>A61P9/00 |
| D,A | GB 2 394 660 A (* NICHE GENERICS LIMITED; * UNICHEM LABORATORIES LIMITED)<br>5 May 2004 (2004-05-05)<br>* abstract *<br>* the whole document *<br>----- | 1-13 | |
| D,A | US 2005/142196 A1 (PATEL ASHISH A ET AL)<br>30 June 2005 (2005-06-30)<br>* the whole document *<br>----- | 1-13 | |
| | | | TECHNICAL FIELDS SEARCHED (IPC) |
| | | | A61K<br>A61P |

The present search report has been drawn up for all claims

| Place of search | Date of completion of the search | Examiner |
|---|---|---|
| Munich | 22 March 2006 | Skjöldebrand, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

2

EPO FORM 1503 03.82 (P04C01)

## ANNEX TO THE EUROPEAN SEARCH REPORT ON EUROPEAN PATENT APPLICATION NO. EP 06 00 2145

This annex lists the patent family members relating to the patent documents cited in the above-mentioned European search report.
The members are as contained in the European Patent Office EDP file on
The European Patent Office is in no way liable for these particulars which are merely given for the purpose of information.

22-03-2006

| Patent document cited in search report | Publication date | Patent family member(s) | Publication date |
|---|---|---|---|
| WO 2005068490 A | 28-07-2005 | SI 21703 A | 31-08-2005 |
| GB 2394660 A | 05-05-2004 | NONE | |
| US 2005142196 A1 | 30-06-2005 | WO 2005007130 A1<br>US 2005009806 A1 | 27-01-2005<br>13-01-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0049658 A
- EP 0308341 A
- EP 1296947 B1
- EP 1294689 A1
- EP 1296948 B1
- WO 2004113293 A
- WO 0115724 A
- EP 280999 A
- WO 9962560 A
- EP 468929 A
- EP 264888 A
- GB 2394660 A
- WO 2005094793 A
- WO 2005068490 A